# EUROPEAN PATENT SPECIFICATION

(11) **EP 0 863 121 B1**
(45) Date of publication and mention of the grant of the patent: **11.10.2000**
(21) Application number: 97902643.2
(22) Date of filing: 10.02.1997
(51) Int. Cl.: C07C 37/04, C07C 39/06, C07C 303/02, C07C 309/30

(54) **PROCESS FOR PREPARING m-HYDROXYALKYLBENZENE**
VERFAHREN ZUR HERSTELLUNG VON M-HYDROXYALKYLBENZOL
PROCEDE DE PREPARATION DE m-HYDROXYALKYLBENZENE

(30) Priority: 24.10.1996 JP 30090396
(43) Date of publication of application: 09.09.1998
(73) Proprietor: TAOKA CHEMICAL COMPANY, LIMITED, Osaka-shi, Osaka 532 (JP)
(72) Inventor: MAKI, Seiji, Higashinada-ku, Kobe-shi, Hyogo 658 (JP); Komiyama, Naoki, Takatsuki-shi, Osaka 569 (JP); KOMIYAMA, Naoki, Takatsuki-shi, Osaka 569 (JP)
(74) Representative: VOSSIUS & PARTNER
(86) International application number: JP9700346
(87) International publication number: WO9817615

(56) References cited:
- JP-A- 8 040 960
- JP-A- 49 110 638
- JP-A- 59 108 730

## Description

The present invention relates to a process for producing a m-hydroxyalkylbenzene and, more particularly, to a process for producing a highly pure m-hydroxyalkylbenzene in high yield and in an industrially advantageous manner.

Various processes are known for the production of m-hydroxyalkylbenzenes. Thus, for example, the process for producing m-hydroxyalkylbenzenes disclosed in Japanese Kokai Publication Sho-59-108730 comprises heating an alkylbenzene or an alkylbenzenesulfonic acid mixture at a temperature within the range of 150°C to 210°C in the presence of sulfuric acid and an inorganic salt to give an alkylbenzenesulfonic acid mixture which is rich in m-isomer content, selectively hydrolyzing alkylbenzenesulfonic acids other than the m-isomer, thereafter rapidly cooling the reaction mixture to 150°C or below by pouring water thereinto and subjecting the alkylbenzenesulfonic acid remaining unhydrolyzed to caustic fusion. The process disclosed in Japanese Kokai Publication Hei-8-40960, which is directed to the preparation of m-hydroxyalkylbenzenes from a mixture of alkylbenzenesulfonic acid isomers by selective desulfonation, removal of the alkylbenzenes formed and caustic alkali fusion, comprises effecting said desulfonation by adding an excessive amount, relative to the amount of evaporating water, of steam to the sulfonic acid mixture while maintaining the temperature of the reaction mixture at a temperature of 160°C or above, until a desired purity relative to said isomer is obtained and until the sulfuric acid content in the reaction mixture is decreased below 40%.

In the process for producing m-hydroxyalkylbenzenes by sulfonation of an alkylbenzene, isomerization, hydrolysis and alkali fusion, to which the present invention is directed, it is an important problem to find a way of easily controlling the above-mentioned step of hydrolysis so that the objective m-hydroxyalkylbenzene can be produced constantly and efficiently.

According to the above-cited reference Japanese Kokai Publication Sho-59-108730, the objective m-hydroxyalkylbenzenes can indeed be produced in high yields as a result of addition of an inorganic salt. However, a procedure for rapid cooling, which comprises adding water, is required after completion of the above-mentioned hydrolysis and, furthermore, it is difficult to properly identify the endpoint of the hydrolysis. Therefore, when evaluated as a commercial constant production process, the process disclosed there has a drawback in such point.

As regards the above-cited reference Japanese Kokai Publication Hei-8-40960, the process disclosed therein, which is likewise directed to an improvement in the step of hydrolysis, comprises adding an excessive amount of steam until the sulfuric acid content in the mixture is decreased below 40%. Said process has a problem in that the high-sulfuric acid content and the addition of steam in excess cause desulfonation of the objective m-alkylbenzenesulfonic acid, leading to a decreased yield of the objective product.

Therefore, these known methods are not fully suited for constant production of desired m-hydroxyalkylbenzenes with high purity, in high yield and in an industrically advantageous manner. Improvements in these respects have been awaited.

### SUMMARY OF THE INVENTION

The present invention provides a process for producing a m-hydroxyalkylbenzene by subjecting an alkylbenzenesulfonic acid mixture to isomerization in the presence of sulfuric acid to give an alkylbenzenesulfonic acid mixture which is rich in m-isomer content, selectively hydrolyzing alkylbenzensulfonic acid isomers other than the m-isomer and subjecting the alkylbenzenesulfonic acid remaining unhydrolyzed to caustic fusion, said process being characterized in that the step of said hydrolysis is started while continuously adding water or steam to the reaction mass at a sulfuric acid content of 18 to 25% by weight and at a temperature within the range of 160°C to 190°C , and said step of hydrolysis is terminated at a final temperature within the range of 162°C to 168°C and at a time when the sulfuric acid content in the reaction mass has reached 32 to 38% by weight.

A characteristic feature of the present invention consists in the understanding that the most important problem to be solved for efficient and constant and industrially advantageous production of objective m-hydroxyalkylbenzenes in the process for producing a m-hydroxyalkylbenzene which comprises sulfonation of an alkylbenzene, isomerization, hydrolysis and alkali fusion is how to control the above-mentioned step of hydrolysis with ease and in the finding of conditions allowing precise indication of the endpoint of such hydrolysis step, in particular.

Particularly, the present inventors found that objective m-hydroxyalkylbenzenes can be produced always constantly with high yield and high purity in an industrially advantageous manner when it only is confirmed that the hydrolysis step mentioned above has proceeded to such an extent that the sulfuric acid content in the reaction mass falls within a specific range in relation to the reaction temperature.

The sulfuric acid content in the reaction mass as referred to herein is the weight percent of sulfuric acid based on the total weight of the reaction mass and can be determined by potentiometric titration.

In the following, the present invention is described in further detail.

The alkylbenzenesulfonic acid mixture to be used in the practice of the present invention can be produced in the conventional manner by sulfonating an alkylbenzene with sulfuric acid. The alkylbenzene to be used here includes toluene, ethylbenzene, isopropylbenzene, 3-methyl-5-isopropylbenzene, 3,5-xylene. In a preferred embodiment of the present invention, ethylbenzene is a preferred species.

The alkylbenzenesulfonic acid mixture obtained in the above manner is isomerized in the presence of sulfuric acid or of sulfuric acid and an inorganic or organic salt to give an alkylbenzenesulfonic acid mixture rich in m-isomer. The sulfuric acid concentration and amount to be used here are not limited to any particular levels but those levels which are conventional may be employed. The isomerization temperature is generally within the range of 180°C to 210°C. The addition of an inorganic or organic salt contributes toward increasing the yield of the objective m-isomer-rich alkylbenzenesulfonic acid and preventing secondary reactions and thus has a very important meaning from the industrial production process viewpoint.

As the inorganic or organic salt suited for use in the practice of the invention, there may be mentioned benzenesulfonic acid salts, alkylbenzenesulfonic acid salts, sodium sulfate, acid sodium sulfate, potassium sulfate, acid potassium sulfate, sodium carbonate, potassium carbonate. In particular, the use of sodium sulfate (Glauber's salt) is preferred. The level of addition of these inorganic or organic salts relative to sulfuric acid is generally 1 to 15 mole percent, preferably 3 to 10 mole percent.

Then, in the step of hydrolysis, other alkylbenzenesulfonic acids than the m-isomer are selectively hydrolyzed and the desulfonation results in the formation of sulfuric acid within the reaction system. Thus, the sulfuric acid content in the reaction mass progressively increases with the progress of hydrolysis.

For selectively hydrolyzing alkylbenzenesulfonic acids other than the m-isomer while keeping the m-isomer intact to thereby produce the m-isomer efficiently in high yield and in a commercially advantageous manner, such conditions as mentioned below must be satisfied:
(1) The sulfuric acid content in the reaction mass at the end of the isomerization reaction should be 18 to 25% by weight, preferably 19 to 23% by weight.
   An excessively high or low sulfuric acid content will lead to a failure to attain the results expected to be obtained from the present invention. Thus, byproducts will be formed, the smooth progress of hydrolysis will be prevented, and the yield of the desired product will be reduced.
(2) The hydrolysis reaction should be carried out at a temperature within the range of 160°C to 190°C .
   The temperature to be employed in the initial phase of the hydrolysis reaction is not critical but should be within the range of 160°C to 190°C , preferably 162°C to 190°C . However, as mentioned later herein, the latter half, in particular the final stage, of the hydrolysis should be carried out within the specified temperature range.
(3) The sulfuric acid content in the reaction mass should be progressively increased by continuously addition of water or steam.
   The expression "continuously adding water or steam" as used herein does not necessarily mean "absolutely continuous addition" but means "addition with the lapse of time". Thus, for example, intermittent continuous addition or a combination of continuous and intermittent additions is also possible. The sulfuric acid content in the reaction system progressively increases with the progress of this hydrolysis (desulfonation). It is important here to allow the progressive sulfuric acid content increase in the reaction mass with the progress of hydrolysis (desulfonation) and discontinue the hydrolysis step before the sulfuric acid content reaches a peak.
(4) The final phase of the hydrolysis reaction should be carried out at a temperature within 162°C to 168°C
   This hydrolysis temperature condition is a very important one to be satisfied for achieving the object of the present invention. An excessively low temperature will lead to failure to smoothly effect the hydrolysis reaction while an excessively high temperature will unfavorably lead not only to byproduct formation but also to hydrolysis of the desired m-alkylbenzenesulfonic acid, in particular.
(5) After allowing the progressive increase of the sulfuric acid content in the reaction mass with the progress of hydrolysis, the hydrolysis step should be terminated when a sulfuric acid content of 32 to 38% by weight, is finally attained.
   This step is one of the most important requirements in the practice of the present invention and means that the sulfuric acid content in the reaction mass must not exceed 38% by weight in relation to the reaction temperature. A sulfuric acid content exceeding 38% by weight tends to cause byproduct formation and/or hydrolysis of the m-alkylbenzenesulfonic acid, thus leading to decreases in m/p ratio, yield of the objective product and quality thereof. On the other hand, if the sulfuric acid content is below 32% by weight at that timepoint, the hydrolysis reaction will proceed not so smoothly as expected.

The p-alkylbenzenesulfonic acid thus selectively hydrolyzed gives the corresponding alkylbenzene and sulfuric acid. The alkylbenzene is distilled off from the reaction system azeotropically with water and generally reused as an additional feed material.

Then, the m-rich alkylbenzenesulfonic acid obtained in the above manner is subjected to the next reaction after addition of water and/or cooling of the reaction mass. This m-rich alkylbenzenesulfonic acid can be stored stably as such and does not require such means as described in the above-cited reference Japanese Kokai Publication Sho-59-108730, for example rapid cooling by addition of water and/or reduction in sulfuric acid concentration. It is of course possible, however, to add water for rapid cooling, which advantageously contributes to reduction of the reaction period, or to add water and perform cooling to lower the sulfuric acid concentration.

Then, the unhydrolyzed alkylbenzenesulfonic acid obtained in the above manner according to the present invention and mainly comprising the m-alkylbenzene sulfonic acid is subjected to caustic fusion by a per se known conventional method, whereby the corresponding m-hydroxyalkylbenzene is produced. Prior to this caustic fusion, that amine extraction described by the present inventors in Japanese Kokai Publication Hei-6-184027 may be applied as a method of pretreatment without any particular limitation.

Thus, for example, when the sulfonation reaction mixture after the hydrolysis step is diluted with water and the extracted with a solution of an amine in toluene, followed by standing and separation into an organic layer and a sulfuric acid layer and further by back extraction of the organic layer with an alkali and the subsequent alkali fusion, the amount of free sulfuric acid can be reduced and the amount of the alkali in the case of alkali salt formation can also be reduced.

The following examples are further illustrative of the present invention but are by no means limitative of the scope of the present invention.

### Example 1

A 6,000-liter reaction vessel was charged with 3,600 kg of 98% sulfuric acid and, then, 300 kg (1.16 moles relative to the sulfuric acid charged) of anhydrous sodium sulfate was added with stirring. Ethylbenzene (2,544 kg) was then added dropwise over 1 hour. The mixture was heated and the temperature was gradually raised to 200°C over 2 hours while the distillate water was drawn off. Isomerization was effected by heating at the same temperature with stirring for 4 hours, whereby a meta/para isomer ratio of 60.1/39.9 was attained. The sulfuric acid content in the reaction mass at that timepoint, as determined by potentiometric titration (hereinafter the same shall apply), was 20.7% by weight.

Then, the internal temperature was lowered to 165°C and, while maintaining that temperature, hydrolysis was effected by continuously blowing an amount of steam which corresponded to 7,800 liters of water into the reaction mass over 12 hours. With the progress of hydrolysis, the sulfuric acid content in the reaction mass progressively increased to a final value of 33.4% by weight. Then, the blowing of steam was discontinued and the hydrolysis reaction was terminated. There was recovered 1,310 kg of ethylbenzene.

The p-/m-ethylbenzenesulfonic acid ratio in the reaction mass after termination of the hydrolysis reaction, as determined by LC analysis, was p/m = 1.8/98.2.

Water (500 liters) was poured into the reaction mass over 30 minutes with external cooling.

Then, the resulting mixture was neutralized to pH 8 by adding 47% caustic soda and, while the mixture was still hot (80°C ), the sodium sulfate was filtered off. The mother liquor was added dropwise to a heated (330°C ) molten mixture of 1,700 kg of caustic soda and 250 kg of caustic potash as placed in a 6,000-liter fusion vessel. After completion of the dropping, the temperature was raised from 330°C to 340°C and stirring was continued at 340°C for 1 hour. The thus obtained fusion reaction mixture was dissolved in 6,000 liters of water, followed by pH adjustment to 7.2 with 35% hydrochloric acid. After phase separation, the layer containing the product phenols was separated and distilled to give crude (94.1% pure) m-ethylphenol. Fractional distillation gave 97.9% pure m-ethylphenol. The yield of m-ethylphenol was 78.7% based on the ethylbenzene consumed.

### Example 2

A 6,000-liter reaction vessel was charged with 3,600 kg of 98% sulfuric acid. Then, 2,544 kg of ethylbenzene was added dropwise over 1 hour. By gradual heating, the temperature was raised to 200°C over 2 hours while the distillate water was drawn off. Isomerization was effected by heating at the same temperature with stirring for 4 hours, whereby a meta/para isomer ratio of 58.4/41.6 was obtained. At this moment, the sulfuric acid content in the reaction mass was 22.8% by weight. The internal temperature was then lowered to 180°C and blowing of steam into the reaction mass was started with stirring at that temperature. After the lapse of 2 hours, the internal temperature was lowered to 165 °C and an amount of steam corresponding to a total of 6,500 liters of water was then continuously blown into the reaction mass with stirring at that temperature over a total of 8 hours to effect hydrolysis. With the progress of hydrolysis, the sulfuric acid content in the reaction mass progressively increased and reached 33% by weight. The blowing of steam was then discontinued and the hydrolysis reaction was terminated. The recovered ethylbenzene amounted to 1,260 kg.

The proportions of p- and m-ethylbenzenesulfonic acid in the reaction mass after the termination of hydrolysis were p/m = 2.6/97.4 as determined by LC analysis.

Then, 500 liters of water was poured over 30 minutes into the reaction mass with external cooling.

The mixture was then neutralized to pH 8 by adding 47% caustic soda. The sodium sulfate was filtered off while the mixture was still hot (80°C ). The mother liquor was added dropwise to a heated (330°C ) molten mixture of 1,700 kg of caustic soda and 250 kg of caustic potash as placed in a 6,000-liter fusion vessel. Thereafter, the temperature was raised from 330°C to 340°C and stirring was continued at 340°C for 1 hour. The thus-obtained fusion reaction mixture was dissolved in 6,000 liters of water, followed by pH adjustment to 7.2 with 35% hydrochloric acid. After phase separation, the layer containing the phenols formed was separated and distilled to give crude (93.2% pure) m-ethylphenol. Fractional distillation gave 96.5% pure m-ethylphenol. The yield of m-ethylphenol was 68.0% on the consumed ethylbenzene basis.

### Example 3

A one-liter flask was charged with 563 g of 98% sulfuric acid and, after addition of 38 g of anhydrous sodium sulfate with stirring, 400 g of ethylbenzene was added dropwise over 1 hour. By gradual heating, the temperature was raised to 200°C over 2 hours while the distillate water was drawn off. Isomerization was effected by heating at the same temperature with stirring for 4 hours, whereby a meta/para isomer ratio of 57.5/42.5 was obtained. At this moment, the sulfuric acid content in the reaction mass was 21.5% by weight as determined by potentiometric titration.

Then, the internal temperature was lowered to 170°C and blowing of steam into the reaction mass was started with stirring at that temperature. After the lapse of 4 hours, the internal temperature was lowered to 165°C and an amount of steam corresponding to a total of 1,250 milliliters of water was continuously blown into the reaction mass over a total of 12 hourswith stirring at that temperature to effect hydrolysis. With the progress of hydrolysis, the sulfuric acid content in the reaction mass progressively increased and reached 35.0% by weight. The blowing of steam was then discontinued and the hydrolysis reaction was thus terminated. In this step, 216 g of ethylbenzene distilled out and was recovered.

The proportions of p- and m-ethylbenzenesulfonic acid in the reaction mass after the termination of hydrolysis were p/m = 1.6/98.4 as determined by LC analysis.

The sulfonation reaction mixture after the termination of hydrolysis was diluted with water. At this timepoint, the sulfuric acid concentration was about 18% by weight. This was extracted, at 60°C, using a 20% dibenzyllaurylamine solution in toluene in such an amount that the mole ratio dibenzyllaurylamine/sulfonic acid in the sulfonation reaction mixture amounted to 1.25. Upon standing, the mixture was separated into an organic layer and an aqueous sulfuric acid layer. The aqueous sulfuric acid layer was recovered for later use in the step of neutralization. The organic layer was back-extracted, at 55°C, using 20% caustic soda in such an amount that the mole ratio caustic soda/dibenzyllaurylamin e-extracted sulfonic acid amounted to 2.2. The alkaline extract was concentrated to such an extent that the sulfonic acid sodium salt concentration became 50%. The concentrate was added dropwise to a heated (330°C ) molten mixture of 216 g of caustic soda and 25 g of caustic potash as contained in a one-liter fusion vessel. Thereafter, the temperature of the flask contents was raised to 340°C and said contents were stirred at that temperature for 60 minutes. The resulting fusion reaction mixture was dissolved in 1,000 ml of water and neutralized to pH 7.2 using the aqueous sulfuric acid separated and recovered in the amine extraction step. The phenols formed were extracted with ether. The subsequent distillation gave crude (95.8% pure) m-ethylphenol. Fractional distillation gave 98.5% pure m-ethylphenol. The yield was 81.0% relative to the ethylbenzene consumed.

### Comparative Examples 1 to 3

For comparison, the procedure of Example 1 was followed while varying, in the hydrolysis step, the sulfuric acid content and the hydrolysis temperature. The results obtained are shown in Table 1.

**Table 1**

| | | Example | | | Comparative Example | | |
|---|---|---|---|---|---|---|---|
| | | 1 | 2 | 3 | 1 | 2 | 3 |
| Addition of inorganic or organic salt | | Yes | No | Yes | No | No | Yes |
| Sulfonic acid content (wt %) | Before hydrolysis | 20.7 | 22.8 | 21.5 | 20.7 | ← | ← |
| | After hydrolysis | 33.4 | 33 | 35.0 | 43.4 | 39.2 | 46.2 |
| Hydrolysis temperature (°C) | Initial stage of hydrolysis | 165 | 180 | 170 | 165 | 180 | 170 |
| | At end of hydrolysis | 165 | 165 | 165 | 165 | 180 | 170 |
| p-/m- ratio in ethylbenzenesulfonic acid | | 1.8 /98.2 | 2.6 /97.4 | 1.6 /98.4 | 3.8 /96.2 | 3.5 /96.5 | 3.6 /96.4 |
| Purity of crude m-ethylphenol (%) | | 94.1 | 93.2 | 95.8 | 90.2 | 91.1 | 90.8 |
| Purity of purified m-ethylphenol (%) | | 97.9 | 96.5 | 98.5 | 93.4 | 94.4 | 94.2 |
| Yield of m-ethylphenol relative to ethylbenzene consumed | | 78.7 | 68.0 | 81.0 | 55.6 | 63.1 | 68.5 |

The process of the present invention makes it possible to produce objective m-hydroxyalkylbenzenes constantly with high purity, in high yield and in an industrially advantageous manner. In particular, the process of the invention enables production of m-ethyl phenol having a high quality as indicated by a purity of at least 96% with a content of the contaminant p-ethylphenol of at most 2.6%.

## Claims

1. A process for producing a m-hydroxyalkylbenzene by subjecting an alkylbenzenesulfonic acid mixture to isomerization in the presence of sulfuric acid to give an alkylbenzenesulfonic acid mixture which is rich in the m-isomer content, selectively hydrolyzing alkylbenzenesulfonic acid isomers other than the m-isomer, and subjecting the alkylbenzenesulfonic acid remaining unhydrolyzed to caustic fusion, characterized in that the step of said hydrolysis is started while continuously adding water or steam to the reaction mass at a sulfuric acid content of 18 to 25% by weight and at a temperature within the range of 160°C to 190°C , and said step of hydrolysis is terminated at a final temperature within the range of 162°C to 168°C and at a time when the sulfuric acid content in the reaction mass has reached 32 to 38% by weight.

2. The process for producing a m-hydroxyalkylbenzene according to Claim 1, wherein the step of hydrolysis is initiated using a reaction mass having a sulfuric acid content of 19 to 23% by weight.

3. The process for producing a m-hydroxyalkylbenzene according to any of Claims 1 or 2, wherein an inorganic or organic salt is caused to coexist in the reaction system in any of said isomerization and hydrolysis steps.

4. The process for producing a m-hydroxyalkylbenzene according to Claim 3, wherein the inorganic or organic salt is selected from among benzenesulfonic acid salts, alkylbenzenesulfonic acid salts, sodium sulfate, acid sodium sulfate, potassium sulfate, acid potassium sulfate, sodium carbonate and potassium carbonate.

5. The process for producing a m-hydroxyalkylbenzene according to Claim 4, wherein the inorganic or organic salt is sodium sulfate.

6. The process for producing a m-hydroxyalkylbenzene according to any of Claims 1 to 5, wherein the m-hydroxyalkylbenzene is m-hydroxyethylbenzene.

## Patentansprüche

1. Verfahren zur Herstellung von m-Hydroxyalkylbenzol, indem ein Alkylbenzolsulfonsäuregemisch in Gegenwart von Schwefelsäure zu einem Alkylbenzolsulfonsäuregemisch mit einem hohen m-Isomergehalt isomerisiert wird, die vom m-Isomer verschiedenenen Alkylbenzolsulfonsäureisomeren selektiv hydrolysiert werden und mit der unhydrolysiert gebliebenen Alkylbenzolsulfonsäure eine Alkalikondensation durchgeführt wird, wobei das Verfahren dadurch gekennzeichnet ist, dass der Hydrolyseschritt gestartet wird, während bei einem Schwefelsäuregehalt von 18 bis 25 Gew.% und einer Temperatur im Bereich von 160 °C bis 190 °C kontinuierlich Wasser oder Wasserdampf zum Reaktionsgemisch gegeben wird, und der Hydrolyseschritt bei einer Endtemperatur im Bereich von 162 °C bis 168 °C und zu einem Zeitpunkt, wenn der Schwefelsäuregehalt im Reaktionsgemisch 32 bis 38 Gew.% erreicht hat, beendet wird.

2. Verfahren zur Herstellung von m-Hydroxyalkylbenzol nach Anspruch 1, wobei der Hydrolyseschritt unter Verwendung eines Reaktionsgemisches mit einem Schwefelsäuregehalt von 19 bis 23 Gew.% gestartet wird.

3. Verfahren zur Herstellung von m-Hydroxyalkylbenzol nach einem der Ansprüche 1 oder 2, wobei ein anorganisches oder organisches Salz in jedem der Isomerisierungs- und Hydrolyseschritte im Reaktionssystem mit vorliegen kann.

4. Verfahren zur Herstellung von m-Hydroxyalkylbenzol nach Anspruch 3, wobei das anorganische oder organische Salz aus Benzolsulfonsäuresalzen, Alkylbenzolsulfonsäuresalzen, Natriumsulfat, saurem Natriumsulfat, Kaliumsulfat, saurem Kaliumsulfat, Natriumcarbonat und Kaliumcarbonat gewählt wird.

5. Verfahren zur Herstellung von m-Hydroxyalkylbenzol nach Anspruch 4, wobei das anorganische oder organische Salz Natriumsulfat ist.

6. Verfahren zur Herstellung von m-Hydroxyalkylbenzol nach einem der Ansprüche 1 bis 5, wobei das m-Hydroxyalkylbenzol m-Hydroxyethylbenzol ist.

## Revendications

1. Procédé pour produire un m-hydroxyalkylbenzène par soumission d'un mélange d'acides alkylbenzènesulfoniques à une isomérisation en présence d'acide sulfurique pour donner un mélange d'acides alkylbenzènesulfoniques qui est riche en l'isomère m, hydrolyse sélective des isomères d'acides alkylbenzènesulfoniques autres que l'isomère m et soumission de l'acide alkylbenzènesulfonique demeurant non hydrolysé à une fusion alcaline, caractérisé en ce que l'étape de ladite hydrolyse est amorcée tandis que de l'eau ou de la vapeur est ajoutée en continu à la masse réactionnelle à une teneur en acide sulfurique de 18 à 25 % en poids et à une température située dans le domaine de 160°C à 190°C, et ladite étape d'hydrolyse est terminée à une température finale située dans le domaine de 162°C à 168°C et à un moment où la teneur en acide sulfurique de la masse réactionnelle a atteint 32 à 38 % en poids.

2. Procédé pour produire un m-hydroxyalkylbenzène selon la revendication 1, où l'étape d'hydrolyse est amorcée au moyen d'une masse réactionnelle ayant une teneur en acide sulfurique de 19 à 23 % en poids.

3. Procédé pour produire un m-hydroxyalkylbenzène selon l'une quelconque des revendications 1 et 2, où un sel inorganique ou organique est amené à coexister dans le système réactionnel dans l'une quelconque desdites étapes d'isomérisation et d'hydrolyse.

4. Procédé pour produire un m-hydroxyalkylbenzène selon la revendication 3, où le sel inorganique ou organique est choisi parmi les sels d'acides benzosulfoniques, les sels d'acides alkylbenzènesulfoniques, le sulfate de sodium, l'hydrogénosulfate de sodium, le sulfate de potassium, l'hydrogénosulfate de potassium, le carbonate de sodium et le carbonate de potassium.

5. Procédé pour produire un m-hydroxyalkylbenzène selon la revendication 4, où le sel inorganique ou organique est le sulfate de sodium.

6. Procédé pour produire un m-hydroxyalkylbenzène selon l'une quelconque des revendications 1 à 5, où le m-hydroxyalkylbenzène est le m-hydroxyéthylbenzène.
